Europäisches Patentamt

European Patent Office

Office européen des brevets

(11) Veröffentlichungsnummer: **0 256 457 B1**

(19)

(12) **EUROPÄISCHE PATENTSCHRIFT**

(45) Veröffentlichungstag der Patentschrift: **15.01.92**

(21) Anmeldenummer: **87111489.8**

(22) Anmeldetag: **08.08.87**

(51) Int. Cl.⁵: **C12N 7/00**

(54) Verfahren zur Züchtung von Granuloseviren.

(30) Priorität: **13.08.86 DE 3627396**

(43) Veröffentlichungstag der Anmeldung:
**24.02.88 Patentblatt 88/08**

(45) Bekanntmachung des Hinweises auf die
Patenterteilung:
**15.01.92 Patentblatt 92/03**

(84) Benannte Vertragsstaaten:
**AT BE CH DE FR GB IT LI NL SE**

(56) Entgegenhaltungen:

**CHEMICAL ABSTRACTS, Band 101, Nr. 25, 17.
Dezember 1984, Seite 432, Zusammenfassung Nr. 226639f, Columbus, Ohio, US; H.G.
MILTENBURGER et al.: "The cellular substrate: a very important requirement for baculovirus in vitro replication", & Z. NATUR-
FORSCH., C: BIOSCI. 1984, 39C(9-10),
993-1002**

**BIOLOGICAL ABSTRACTS, Band 78, 1984,
Zusammenfassung Nr. 18213, Biological Abstracts, Inc., Philadelphia, US; D.M. GLEN et
al.: "Production and field evaluation of coding moth granulosis virus for control of
Cydia pomonella in the UK", & ANN APPL**

**BIOL 104(1): 87-98. 1984**

(73) Patentinhaber: **HOECHST AKTIENGESELL-
SCHAFT
Postfach 80 03 20
W-6230 Frankfurt am Main 80(DE)**

(72) Erfinder: **Gröner, Albrecht, Dr.
Fasanenweg 6
W-6104 Seeheim-Jugenheim(DE)**
Erfinder: **Knauf, Werner, Dr.
Im Kirschgarten 24
W-6239 Eppstein/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Granuloseviren, zu der Familie Baculoviridae gehörend, vermehren sich nach oraler Aufnahme durch empfindliche Insektenlarven in verschiedenen Organen und Geweben dieser Insekten. Cytopathogene Effekte führen zum Verenden der Insektenlarven.

Das Granulosevirus des Apfelwicklers [Cydia (= Laspeyresia = Carpocapsa) pomonella L] aus der Familie der Tortriciden) wurde 1963 in Berkeley, Kalifornien aus Apfelwicklerlarven isoliert [TANADA, Y.: J. Insect Pathol. 6, 378, 1984]. Seine Kurzbezeichnung lautet "CpGV." CpGV ist zur selektiven Bekämpfung des Apfelwicklers im Rahmen des integrierten Pflanzenschutzes im Obstbau hervorragend geeignet [HUBER, Mitt. dtsch. Ges. allg. angew. Ent. 4, 55, 1983].

Die Produktion des CpGV erfolgt in Apfelwicklerlarven durch Infektion der Larven im letzten Larvenstadium mit dem Granulosevirus und nachfolgender Reinigung der Viren aus den Larvenkadavern [z.B.: HUBER, J.: Mitt. dtsch. Ges. allg. angew. Ent. 2, 141, 1981; GLEN, D.M. & PAYNE, C.C.: Ann. appl. Biol. 104, 87, 1984].

Aufgrund der sehr hohen Virulenz des CpGV für Apfelwicklerlarven muß die Massenzucht des Apfelwicklers unter semisterilen Bedingungen erfolgen, um eine Durchseuchung der Zuchtansätze mit CpGV zu verhindern. Diese semisterile Zucht ist sehr arbeits- und kostenintensiv.

Es wurde nun überraschenderweise gefunden, daß sich eine CpGV-Produktion auch in anderen Tortricidenarten in vorteilhafter Weise durchführen läßt.

Gegenstand der vorliegenden Erfindung ist daher ein Verfahren zur Züchtung des Cydia pomonella-Granulosevirus, dadurch gekennzeichnet, daß man das Virus in Larven von Tortricidenarten, die gegenüber diesem Virus einen um den Faktor 5 bis 100000, vorzugsweise 10 bis 5000 höheren $LD_{50}$-Wert als Apfelwicklerlarven aufweisen, vermehrt.

Bevorzugt wird das Verfahren in Larven der Unterfamilie Olethreutinae, beispielsweise in Larven von Grapholita molesta, Rhyacionia buoliana, Cydia nigricana oder Cryptophlebia leucotretra, insbesondere in Larven von Cryptophlebia leucotretra Meyr durchgeführt. Für letztere Spezies war bisher keine Pathogenität gegenüber CpGV bekannt.

Das erfindungsgemäße Verfahren wird unter nicht-semisterilen, d.h. normalen hygienischen Bedingungen durchgeführt. Für die Züchtung der Tortriciden-Larven werden Temperaturen gewählt, die über den für bekannte CpGV-Zuchtverfahren üblichen Temperaturen liegen. Hierdurch kann das Produktionsverfahren für CpGV erheblich abgekürzt werden.

Die Zucht von Olethreutinae-Arten findet auf semisynthetischem Nährmedium, bestehend aus einer Kohlenstoffquelle wie Mais- oder Bohnenmehl, einer Protein-, Vitamin- und Spurenelementquelle wie Weizenkeime und Bier- bzw. Futterhefe, Ascorbinsäure, Fungistatica sowie Geliermittel und/oder wasserbindenden Substanzen wie Agar-Agar, bei 20 bis 34°C, bevorzugt bei 26 bis 30°C statt. Unter diesen Bedingungen dauert die Entwicklung einer Generation 20 bis 50 Tage.

Die Infektion der Larven mit CpGV erfolgt in frühen Larvenstadien, bevorzugt im 2. oder 3. Stadium, durch Kontamination der Nährmedienoberfläche. Die Insektenlarven werden dann bei den obengenannten Temperaturen gehalten. 5 bis 14 Tage, bevorzugt 6 bis 9 Tage nach der Infektion werden die virösen Kadaver aufgearbeitet und das CpGV in bekannter Weise daraus isoliert.

Die Erfindung wird durch nachstehendes Beispiel näher erläutert.

Produktion des CpGV in Cryptophlebia leucotretra

Frisch geschlüpfte Falter von C. leucotretra (Geschlechterverhältnis etwa 1 : 1) wurden in mit Schaumstoff ausgeschlagene Eiablagekäfige überführt, deren obere Öffnung mit Klarsichtfolie abgedeckt ist. Die Falter legten Eier auf die Klarsichtfolie ab, die täglich gewechselt wurde. Als Nahrung für die Falter diente Wasser, zum Teil wurde Hefeextrakt zugegeben. Die Inkubationstemperatur betrug 24 bis 30°C.

Von den aus Eiern schlüpfenden Larven wurden jeweils 100 Larven auf je 200 ml eines semisynthetischen Nährmediums, bestehend aus 20 g Agar-Agar, 140 g Maisgrieß, 35 g Weizenkeime, 38 g Bierhefe, 5 g Ascorbinsäure, 2,3 g Benzoesäure, 1,8 g p-Hydroxybenzoesäure, und 760 g Wasser pro kg Medium, gegeben. Bei einer Inkubationstemperatur von 28°C wurde nach 6 Tagen zum Zeitpunkt, als sich die Larven Ende des zweiten bis Anfang des dritten Larvenstadiums befanden, die Nährmedienoberfläche mit einer Granulosevirussuspension kontaminiert. Man stellte auf eine Konzentration von 2,5 x $10^6$ Viren/cm² ein. Nach weiteren 6 bis 9 Tagen wurden die virösen Kadaver aus dem Nährmedium abgesaugt und die Granula durch differenzielle Zentrifugation gereinigt. Die Ausbeute betrug 1,0 bis 1,2 x $10^{12}$ Viruspartikel (Granula) pro 200 ml Medium.

## Patentansprüche

1. Verfahren zur Züchtung des Cydia pomonella-Granulosevirus (CpGV), dadurch gekennzeichnet, daß man das Virus in Larven von Tortricidenarten, die gegenüber diesem Virus einen um den Faktor 5 bis 100000 höheren $LD_{50}$-

Wert als Apfelwicklerlarven besitzen, vermehrt.

2. Verfahren gemäß Anspruch 1, dadurch gekennzeichnet, daß es in Larven von Tortricidenarten durchgeführt wird, die einen um den Faktor 10 bis 5000 höheren $LD_{50}$-Wert als Apfelwicklerlarven besitzen.

3. Verfahren nach Ansprüchen 1 oder 2, dadurch gekennzeichnet, daß es in Larven der Unterfamilie Olethreutinae durchgeführt wird.

4. Verfahren nach einem oder mehreren der Ansprüche 1, 2 oder 3, dadurch gekennzeichnet, daß es in Larven von Grapholita molesta, Rhyacionia buoliana, Cydia nigricana oder Cryptophlebia leucotretra durchgeführt wird.

5. Verfahren gemäß einem oder mehreren der Ansprüche 1 bis 4, dadurch gekennzeichnet, daß es in Larven von Cryptophlebia leucotretra durchgeführt wird.

## Claims

1. A process for growing Cydia pomonella granulosis virus (CpGV), which comprises propagating the virus in larvae of Tortricidae species which have a $LD_{50}$ for this virus which is a factor of 5 to 100,000 higher than that of codling moth larvae.

2. The process as claimed in Claim 1, which is carried out in larvae of Tortricidae species which have a $LD_{50}$ which is a factor of 10 to 5,000 higher than that of codling moth larvae.

3. The process as claimed in Claim 1 or 2, which is carried out in larvae of the subfamily Olethreutinae.

4. The process as claimed in one or more of Claims 1, 2 or 3, which is carried out in larvae of Grapholita molesta, Rhyacionia buoliana, Cydia nigricana or Cryptophlebia leucotretra.

5. The process as claimed in one or more of Claims 1 to 4, which is carried out in larvae of Cryptophlebia leucotretra.

## Revendications

1. Procédé pour cultiver le virus de la granulose de Cydia pomonella (CpGV), procédé caractérisé en ce qu'on multiplie le virus dans des larves d'espèces de tortricidés qui présentent, pour ce virus, une valeur $DL_{50}$ supérieure d'un facteur de 5 à 100.000 à celle de larves de la pyrale des pommes.

2. Procédé selon la revendication 1 caractérisé en ce qu'il est exécuté dans des larves d'espèces de tortricidés qui ont une valeur $DL_{50}$ supérieure d'un facteur de 10 à 5.000 à celle de larves de la pyrale des pommes.

3. Procédé selon l'une des revendications 1 et 2, caractérisé en ce qu'il est exécuté dans des larves de la sous-famille des Oléthreutinés.

4. Procédé selon l'une quelconque des revendications 1 à 3, caractérisé en ce qu'il est exécuté dans des larves de Grapholita molesta, de Rhyacionia buoliana, de Cydia nigricana ou de Cryptophlebia leucotretra.

5. Procédé selon l'une quelconque des revendications 1 à 4, caractérisé en ce qu'il est exécuté dans des larves de Cryptophlebia leucotretra.